# EUROPEAN PATENT SPECIFICATION

(11) **EP 1 198 289 B1**
(45) Date of publication and mention of the grant of the patent: **17.03.2004**
(21) Application number: 00931236.4
(22) Date of filing: 15.05.2000
(51) Int. Cl.: B01J 19/02, C07C 303/06, C07C 309/31

(54) **SULPHONATION PROCESS AND INHIBITION OF BUILD-UP IN REACTION VESSELS**
VERFAHREN ZUR SULFONIERUNG SOWIE VERHINDERUNG VON ABLAGERUNGEN IN REAKTORBEHÄLTERN
PROCEDE DE SULFONATION ET EMPECHEMENT DE L'ACCUMULATION DE DEPOTS DANS DES RECIPIENTS DE REACTION

(30) Priority: 21.05.1999 GB 9911948
(43) Date of publication of application: 24.04.2002
(73) Proprietor: UNILEVER PLC, London EC4P 4BQ (GB); UNILEVER N.V., 3013 AL Rotterdam (NL)
(72) Inventor: CHADWICK, Paul, Unilever Research Vlaardingen, 3133 AT Vlaardingen (NL); STEWART, John, Unilever Research Port Sunlight, Wirral, Merseyside CH63 3JW (GB)
(74) Representative: Elliott, Peter William
(86) International application number: PCT/EP2000/004433
(87) International publication number: WO 2000/071244

(56) References cited:
- EP-A- 0 327 202
- US-A- 3 839 391
- US-A- 4 098 972

## Description

### TECHNICAL FIELD

The present invention relates to the inhibition of the formation of contaminating material in a reaction vessel occurring as a result of a sulph(on)ation reaction. More particularly, the present invention relates to the pretreatment of reaction vessels with a manganese compound.

### BACKGROUND TO THE INVENTION

Sulphation and sulphonation reactions are commonly used in the detergent industry in the manufacture of sulphated and sulphonated anionic surfactants. The sulphonation and sulphation processes are usually similar, and in many cases, the same plant can be used for either. So as to avoid tedious repetition, hereinafter the term "sulphonation" includes sulphation, unless otherwise specified.

Sulphonation reactions, especially when employed on an industrial scale, tend to experience problems over time with the build up of contaminating material in the reaction vessel. This is probably due, at least in part, to the high level of heat generated and the subsequent requirement for active cooling systems. Such problems include contaminating material being deposited on the walls of the reaction vessel or other parts, such as connecting piping, wetted by the reaction materials. As deposition continues, there is often a decrease in product yield and a deterioration in product quality as the reaction fails to go to completion and the product becomes contaminated by the deposited material. Furthermore, in reactions involving heat transfer there is often a lowering of, for example, the cooling capacity of the reactor vessel.

The formation of charred material is a particular problem in sulphonation reactions. The exact nature of the charred material is not known, however, without being limited by theory, it is believed to consist of polysulphonated polycyclic aromatic compounds resulting from sulphonation of oxidised and oligomerised materials formed as a result of overheating caused by the rapid evolution of heat from the exothermic sulphonation reaction.

One of the most commonly used sulphonation reactor vessels, used essentially in the manufacture of anionic surfactants, is that of the falling film type, which works on the basis of a continuous thin film of liquid falling down the inner surface of a tube. Falling film reactors are particularly prone to and sensitive to the formation of contaminating material when operated non-optimally and when oversulphonation takes place. Even a small amount of contaminating material on the inner surface of a tube can break the continuity of the thin film leading to areas of overheating in the film, thus exacerbating the formation of contaminating charred material. This can eventually lead to the tubes becoming completely blocked.

Hence, in sulphonation reactions where deposition is a problem, plants need to be shutdown and deposited material removed on a regular basis. The costs involved in cleaning in terms of production downtime and labour are considerable.

We have now surprisingly found that pretreating a reaction vessel with a manganese compound inhibits the formation and/or deposition of contaminating material resulting from a sulphonation reaction. By pretreating reaction vessels with a source of a manganese compound or compounds, we have found that sulphonation reaction vessels remain "clean" for a significantly longer period. Thus the number of production shutdowns required for cleaning is significantly reduced leading to considerable time and cost savings. Furthermore, we have found that acceptable reaction product colour and quality can be maintained for a longer period of continuous use of the reaction vessel before the reaction vessel needs cleaning.

### PRIOR ART

EP 327202 (Shin-Etsu) describes the use of manganese compounds to pretreat reactors so as to inhibit polymer scale deposition occurring during polymerisation of ethylenically unsaturated monomers.

### DEFINITION OF THE INVENTION

Thus, in a first aspect, the present invention relates to a method of inhibiting the build-up of contaminating material in a sulphonation reaction vessel, which comprises treating the surfaces of the reaction vessel exposed to the sulphonation reaction mixture with a pretreatment fluid containing a source of manganese whereby a coating comprising manganese dioxide is formed on these surfaces.

In a second aspect, the present invention relates to the use of a manganese source to generate a coating comprising manganese dioxide on the surface of a sulphonation reaction vessel.

In a third aspect, the present invention relates to a sulphonation process which comprises treating a sulphonatable feedstock with a source of sulphur trioxide, characterised in that the process is carried out in a reaction vessel of which the surfaces exposed to the sulphonation reaction mixture have a coating comprising manganese dioxide, as set fourth in the appended claims.

In a fourth aspect, the present invention relates to a sulphonation reaction vessel wherein the surfaces exposed to the sulphonation reaction mixture have a coating comprising manganese dioxide.

### DETAILED DESCRIPTION OF THE INVENTION

The present invention is based on the use of a pretreatment fluid containing a source of manganese to generate a coating comprising manganese dioxide on those surfaces of a sulphonation reactor that will be exposed to the reaction mixture.

The source of manganese can be any manganese compound which is capable of forming a coating comprising manganese dioxide on the surface of a reaction vessel.

The coating is in effect a thin layer, solid state passive film. The presence of manganese dioxide in the coating, and hence the suitability of the manganese source for a particular reaction vessel, can be readily assessed using in situ X-ray absorption near-edge spectroscopy (XANES). The XANES technique can be used to establish if a small sample of the material from which a particular reaction vessel is made is coated by the manganese source. The XANES technique is a common analysis technique used in corrosion science in the study of passive films. The technique involves exposing a target specimen to monochromatised X-rays of sufficient energy to excite a core electron in the specimen to an unoccupied valence level or to the continuum. The absorption of the incident X-rays produces an absorption edge in the corresponding spectrum and the position of the absorption edge is characteristic of the absorbing atom. By comparing data with that of model standard compounds, the oxidation state of absorbing atoms can be determined from the position of the absorption edge. Precise details of how to carry out the technique will be known to those skilled in the art and can be found in the literature, for example, A.J. Davenport & M. Sansone, (1995), "High resolution in-situ XANES Investigation of the nature of passive films on iron in a pH 8.4 borate buffer", J. Electrochem. Soc. Vol. 142, pages 725-730.

Pretreating the reaction vessel leads to the formation of a coating comprising manganese dioxide on the surface of the reaction vessel wetted by the pretreatment fluid. Sources capable of forming manganese dioxide can also often lead to the formation of other oxides of manganese. The present invention is therefore not limited to sources of manganese which lead to coatings only containing manganese dioxide. Other oxides of manganese may also be present. However, it is preferred that manganese dioxide predominates.

In addition, the manganese source may lead to oxidation of constituents inherent in the surface material of the reaction vessel. For example, if the reaction vessel is made of stainless steel, various iron oxides and/or chromium oxides may result and may form part of the manganese dioxide-containing solid state thin film.

Solid state thin films are typically ultra-thin, e.g. no more than 20 nm and often in the range of from 1 to 10 nm. Depth profiles of solid state films can be measured using various electron spectroscopy techniques such as X-ray photoelectron spectroscopy (XPS) and Auger Electron Spectroscopy (AES). See, for example, J.F. Watts, (1990), "An Introduction to Surface Analysis by Electron Spectroscopy", in Microscopy Handbooks, Vol. 22, and D. Briggs & M.P. Seah, (1990), "Practical Surface Analysis", Vol. 1.

Preferably, the source of manganese is water-soluble.

Preferably, the source of manganese is a permanganate salt such as potassium, sodium or ammonium.

The source of manganese can be a single chemical compound source or can be derived from a mixture of chemical compounds.

The reaction vessel is pretreated by exposing it to a fluid containing one or more of the manganese sources. The manganese source(s) is either dissolved or suspended in the solvent, preferably dissolved. The solvent used to dissolve or suspend the manganese source(s) may be water or a water-miscible organic solvent such as alcohols, esters or ketones, or a mixture of two or more of these solvents.

The fluid containing the manganese source(s) (i.e. the pretreatment fluid) is introduced in any convenient manner to the reaction vessel. The pretreatment fluid may simply be poured in or it may, for example, be sprayed in.

Preferably, the reaction vessel is treated with the pretreatment fluid at a temperature of greater that 5°C, more preferably greater than 10°C, yet more preferably greater than 20°C. Preferably the temperature is not greater than 100°C. If the manganese source is soluble, then preferably a temperature is employed at which substantially all the manganese source is in solution. A highly preferred temperature range is from 50 to 100°C, preferably from 70 to 100°C, more preferably from 80 to 90°C.

The pretreatment fluid can either be preheated to an appropriate temperature prior to introduction to the reaction vessel or can be heated once introduced to the reaction vessel by, for example, use of a heated jacket.

The pretreatment fluid is suitably kept in continuous contact with the reaction vessel for at least 10 minutes, preferably for at least 15 minutes, more preferably for at least 30 minutes and most preferably for at least 60 minutes.

In a preferred embodiment, the concentration of the manganese source(s) in the pretreatment fluid is at least 0.1, preferably at least 0.2, more preferably at least 0.5, and most preferably at least 0.75% by weight. Furthermore, the concentration of the manganese source(s) in the pretreatment fluid is preferably no more than 10, more preferably no more than 5, most preferably less no more than 3% by weight. It should be noted that in specifying any range of concentration, any particular upper concentration can be associated with any particular lower concentration.

In a preferred embodiment, the manganese source is water-soluble such as potassium permanganate and the pretreatment fluid is a 0.5 to 3% by weight, for example 2% by weight, aqueous solution.

Of course, by employing the techniques described above such as XANES, AES and/or XPS, it will be evident to the skilled person if a treatment time of less than 10 minutes or significantly in excess of 60 minutes may be employed. Likewise, it will also be evident if a concentration of manganese source in the pretreatment fluid of less than 0.1 or greater than 10% by weight may be usefully employed.

Once the reaction vessel has been treated for an appropriate period of time, excess pretreatment fluid is generally discharged from the reaction vessel. The reaction vessel is then generally washed with water or another appropriate solvent to remove any manganese source and/or solvent which does not form part of the coating.

It is highly preferred that prior to the pretreatment process, the reaction vessel be very clean, e.g. visually "spotlessly" clean.

The method of the invention is applicable to reaction vessels made of various kinds of materials such as ferrous alloys, for example stainless steel, and nickel-based alloys, for example Hastelloy (Trade mark). In particular, the present invention is applicable to stainless steel reaction vessels.

A reaction vessel may be treated more than once, although preferably not at each shutdown.

Sulphonation reactions are regularly used in the manufacture of cleaning components, especially anionic surfactants, which may be employed in personal wash or laundry detergent compositions. It is generally undesirable for even very low amounts of heavy metal compounds to be present in such compositions as they can lead to undesirable interactions with certain components of detergent compositions. See for example WO 92/06163 (Procter & Gamble). Surprisingly, the levels of heavy metal compounds in the sulphonated product produced by the present process are very low, and well below those reported as having a deleterious effect on, for example, laundry detergent powders.

The present invention has been found to be particularly useful at inhibiting the formation of contaminating material in sulphonation processes involved in the manufacture of anionic surfactants. Thus, in a preferred embodiment, the present invention provides a process for the sulphonation of a sulphonatable feedstock which is a precursor of a sulphonate- or sulphate-type anionic surfactant.

Suitable anionic surfactants, the production of which can benefit from the present invention are well-known to those skilled in the art. Examples include alkylbenzene sulphonates, particularly linear alkylbenzene sulphonates having an alkyl chain length of C₈-C₁₅; primary and secondary alkyl sulphates, particularly C₁₂-C₁₅ primary alkyl sulphates; alkyl ether sulphates; olefin sulphonates; alkyl xylene sulphonates; dialkyl sulphosuccinates; and fatty acid ester sulphonates.

The feedstocks which are sulphonated are well-known to those skilled in the art. According to a preferred embodiment of the invention the sulphonatable feedstock is a linear alkylbenzene.

The present invention has been found to be especially beneficial in inhibiting the deposition of overheated and/or charred material in sulphonation reaction vessels, especially in falling film reaction vessels used in the manufacture of anionic surfactants. Such reactor vessels work on the basis of a thin film of organic material flowing down the inner surface of a set of tubes with a gaseous sulphur trioxide/air stream flowing cocurrently down the inside of the tubes. Sulphur trioxide is absorbed by the liquid and reacts to form the sulphonated product. The heat of reaction is removed by coolant applied to the surface of the tubes. The reactors can be either tubular or annular in design.

In a preferred embodiment, the present invention provides a sulphonation reaction vessel of the falling film type in which the surfaces exposed to the sulphonation reaction mixture have a coating comprising manganese dioxide.

Furthermore, by coating sulphonation reactors according to this invention, acceptable product colour is obtained over a longer period of continuous use of the reactor.

Reference herein to surfaces of reaction vessel includes all those parts of the reaction apparatus wetted by, exposed to vapours of or which come in any way into contact with the sulphonation reaction starting, intermediate or product materials or any mixture thereof.

The invention is now further illustrated by the following non-limiting examples:

### EXAMPLES

### Example 1

In Example 1, a 120 stainless steel tube falling film sulphonation reactor was used to sulphonate a linear alkylbenzene using sulphur trioxide.

The tubes of the reactor were cleaned using normal factory procedures and endoscopically examined to ensure they were visually "spotlessly" clean. If necessary, they were recleaned. Sulphonation was then carried out as normal at a standard throughput rate for a continuous period of 45 days. After this time, product colour monitoring indicated the reactor needed cleaning. Endoscopic visual inspection revealed 22 of the 120 tubes to be blocked due to the build up of contaminant material.

The tubes were cleaned using normal factory procedures. Endoscopic examination was performed to ensure the tubes were "spotlessly" visually clean. If necessary, cleaning was repeated.

The reaction vessel was then treated with a potassium permanganate source. The tubes were plugged (water tight) ready to receive the pretreatment fluid. Each tube was filled with a 1% w/w aqueous potassium permanganate solution at 90-100°C. The pretreatment fluid remained in the tubes for 1 hour and was then discharged. The tubes were flushed with water until all the pretreatment fluid had been removed. Pretreatment with the potassium permanganate solution followed by flushing was repeated 3 times for each tube.

Once pretreatment was complete, the reactor was thoroughly washed through using the normal factory procedure and endoscopic examination repeated.

Sulphonation was carried out as normal at a standard throughput rate for a continuous period of 50 days. At this point, monitoring of product colour indicated the reactor needed cleaning. The reactor was shutdown and cleaned following normal factory procedure, without any further manganese pretreatment.

The reactor was then restarted and run as normal at a standard throughput rate for a continuous period of 56 days. Monitoring the colour of the product indicated the reactor needed cleaning at this point. Endoscopic Visual inspection of the tubes prior to cleaning showed that only 5 tubes were blocked by contaminating material.

The results indicate a significant reduction in the level of contaminating material, during normal operation, in the vessel pretreated with the manganese source as compared with the untreated vessel. This allowed the sulphonation plant to be operated for a longer period in between wash outs.

Furthermore, there was an improvement in product colour observed over time from the pretreated reactor vessel as compared with the untreated vessel.

### Example 2

The effect of pretreatment with a manganese source was investigated on a falling film sulphonation reactor vessel that had been in use for over ten years. The tubes were cleaned with hot water and examined with a video endoscope. 6 tubes were then treated with a 1% w/w aqueous potassium permanganate solution and 6 tubes with a 2% w/w aqueous potassium permanganate solution.

2 of the tubes treated with the 2% w/w solution were pretreated by recirculating the permanganate solution via a pump and stainless steel tank at a temperature of 83-87°C for 1 hour. The remaining tubes were filled with potassium permanganate solution at 90°C. The temperature of the solution decayed to 65°C during the course of the 1 hour treatment. All the tubes were subsequently flushed through with water.

A further endoscopic inspection was carried out posttreatment to ensure the treated tubes were clean.

The reactor was then used as normal for some 4 months before the next cleaning operation. Prior to cleaning, the tubes were re-inspected by video endoscope which revealed that tubes which had been pretreated were cleaner than untreated tubes. The tubes treated with 2% w/w permanganate solution were clean along their entire length.

There was no clear benefit observed in pretreating tubes with a constant flow of manganese solution flow at a constant temperature versus the use of a stagnant permanganate solution at a temperature which was allowed to decay.

## Claims

1. A sulphonation process which comprises treating a sulphonatable feedstock with a source of sulphur trioxide, **characterised in that** the process is carried out in a reaction vessel of which the surfaces exposed to the sulphonation reaction mixture have a coating comprising manganese dioxide.

2. A sulphonation process according to claim 1 in which the coating comprising manganese dioxide is formed by treating the surfaces of the reaction vessel exposed to the sulphonation reaction mixture with a pre-treatment fluid containing a source of manganese.

3. A sulphonation process according to claim 2 in which the pre-treatment fluid is an aqueous solution of a water soluble manganese salt.

4. A sulphonation process according to claim 3 in which the water soluble manganese salt is a permanganate salt, preferably potassium permanganate.

5. A sulphonation process according to any preceding claim in which the coated surfaces of the reaction vessel are stainless steel.

6. A sulphonation process according to any preceding claim in which the sulphonation reaction is carried out in a falling film reactor.

7. A sulphonation process according to any preceding claim in which the sulphonatable feedstock is a precursor of a sulphonate- or sulphate-type anionic surfactant.

8. A sulphonation process according to claim 7 in which the sulphonatable feedstock is a linear alkylbenzene.

## Patentansprüche

1. Sulfonierungsverfahren, umfassend das Behandeln eines sulfonierbaren Ausgangsmaterials mit einer Schwefeltrioxidquelle, **dadurch gekennzeichnet, dass** das Verfahren in einem Reaktionsgefäß durchgeführt wird, dessen dem Sulfonierungsreaktionsgemisch ausgesetzte Oberflächen eine Mangandioxid aufweisende Beschichtung aufweisen.

2. Sulfonierungsverfahren nach Anspruch 1, bei dem die Mangandioxid aufweisende Beschichtung durch Behandeln der dem Sulfonierungsreaktionsgemisch ausgesetzten Oberflächen des Reaktionsgefäßes mit einer eine Manganquelle enthaltenden Vorbehandlungsflüssigkeit gebildet wird.

3. Sulfonierungsverfahren nach Anspruch 2, bei dem die Vorbehandlungsflüssigkeit eine wässrige Lösung eines wasserlöslichen Mangansalzes ist.

4. Sulfonierungsverfahren nach Anspruch 3, bei dem das wasserlösliche Mangansalz ein Permanganatsalz, bevorzugt Kaliumpermanganat ist.

5. Sulfonierungsverfahren nach einem der vorstehenden Ansprüche, bei dem die beschichteten Oberflächen des Reaktionsgefäßes aus rostfreiem Stahl sind.

6. Sulfonierungsverfahren nach einem der vorstehenden Ansprüche, bei dem die Sulfonierungsreaktion in einem Fallfilm-Reaktor durchgeführt wird.

7. Sulfonierungsverfahren nach einem der vorstehenden Ansprüche, bei dem das sulfonierbare Ausgangsgemisch ein Vorläufer eines anionischen Tensids vom Sulfonat- oder Sulfattyp ist.

8. Sulfonierungsverfahren nach Anspruch 7, bei dem das sulfonierbare Ausgangsgemisch ein lineares Alkylbenzol ist.

## Revendications

1. Procédé de sulfonation qui comprend l'étape consistant à traiter une charge sulfonable avec une source de trioxyde de soufre, **caractérisé en ce que** le procédé est effectué dans un récipient de réaction dont la surface exposée au mélange réactionnel de sulfonation a un revêtement comprenant du dioxyde de manganèse.

2. Procédé de sulfonation selon la revendication 1, dans lequel le revêtement comprenant du dioxyde de manganèse est formé en traitant les surfaces du récipient de réaction exposées au mélange réactionnel de sulfonation avec un fluide de prétraitement contenant une source de manganèse.

3. Procédé de sulfonation selon la revendication 2, dans lequel le fluide de prétraitement est une solution aqueuse d'un sel de manganèse soluble dans l'eau.

4. Procédé de sulfonation selon la revendication 3, dans lequel le sel de manganèse soluble dans l'eau est un sel de permanganate, de préférence du permanganate de potassium.

5. Procédé de sulfonation selon l'une quelconque des revendications précédentes, dans lequel les surfaces revêtues du récipient de réaction sont en acier inoxydable.

6. Procédé de sulfonation selon l'une quelconque des revendications précédentes, dans lequel la réaction de sulfonation est effectuée dans un réacteur à flot tombant.

7. Procédé de sulfonation selon l'une quelconque des revendications précédentes, dans lequel la charge sulfonable est un précurseur de tensioactif anionique de type sulfonate ou de type sulfate.

8. Procédé de sulfonation selon la revendication 7, dans lequel la charge sulfonable est un alkylbenzène linéaire.
